# EUROPEAN PATENT APPLICATION

(11) **EP 4 141 887 A1**
(43) Date of publication of application: **01.03.2023**
(21) Application number: 21194045.7
(22) Date of filing: 31.08.2021
(51) Int. Cl.: G16H 80/00

(54) **METHODS, SYSTEMS, COMPUTING DEVICES FOR DIGITAL COOPERATION**

(71) Applicant: Siemens Healthcare GmbH, 91052 Erlangen (DE)
(72) Inventor: Barrios Romero, Diego, 27711 Osterholz-Scharmbeck (DE); Brachmann, Christoph, 28832 Achim (DE); Höfener, Henning, 28832 Achim (DE); Kohle, Sven, 91056 Erlangen (DE); Lippok, Svenja, 91080 Uttenreuth (DE); Neugebauer, Mathias, 28215 Bremen (DE); Ritter, Felix, 28203 Bremen (DE); Schildhaus, Hans-Ulrich, 37077 Göttingen (DE)

(57) **Abstract**

In one aspect, the present invention relates to a method (10) for digital cooperation for a group of collaborating computing devices by providing access to a health-related application persisting an application state for a medical assessment, comprising the method steps of:
- Executing (S11) a health-related application instance on a sending computing device (220) providing means for accessing and controlling the executed health-related application;
- Bookmarking (S12) an application state of an executed health-related application instance on the sending computing device (220) to be output and shared within the group of collaborating computing devices;
- Storing (S13) by a backend computing device (300) the bookmarked application state of an application instance, the application instance being executed on the sending computing device (200);
- Generating (S14) by the backend computing device (300) on the basis of the stored bookmarked application state a container as a data structure including health-related application data;
- Generating (S15) by the backend computing device (300) a link referring to the generated container;
- By the backend computing device (300) or by the sending computing device (220): Initiating (S16a, S16b) a sending of the generated link to at least one further selected computing device (230) of the collaborating computing devices; and
- Receiving (S17) the link by the at least one further selected computing devices (230);
- Activating (S18) the received link by the at least one further selected computing device (230) for providing access to the health-related application data and thereby processing an instance of the container; and
- Storing (S19) by the backend computing device (300) any processed instance of the container and making it available for further collaboration within the group of collaborating devices.

## Description

The present invention generally relates to methods, systems, a computing device, and a backend computing device for digital cooperation for a group of collaborating devices by providing access to a health-related application persisting an application state for a medical assessment. Further, the present invention relates to a computer program product.

An important component of a holistic medical treatment in the health sector is the constantly improved cooperation of different medical professions. A key for success is the interdisciplinary cooperation including the coordination of the expertise of all healthcare professions. Through this cooperation, patients benefit from high quality on the one hand and the public health system from efficiency on the other. In particular, a medical diagnosis can be performed more precise and efficient.

However, for a common and interdisciplinary assessment of a complex medical issue, it is necessary to communicate a compilation of all relevant information in order to exchange the assessment of different medical professions (experts) with the aim of making a profound decision on further diagnostic or therapeutic treatments. For this purpose, each expert needs the essential summarized information of his own or other disciplines that may be prepared by a machine, e.g. by an artificial intelligence based system, or human experts, as well as a point of entry into the information with the possibility of interactively re-viewing the given information and to consolidate the total amount of available information for the requested medical issue, in order to get his/her own analysis of the issue.

The result of this analysis has to be represented and made available to other experts or protocol systems. In addition, a representation that may be reduced in selection or simplified is an important basis for a communication of the essential information to an attending physician or to the patient himself. The compilation of the relevant data per case is very time-consuming and the common use with colleagues is tedious, because everybody stores the relevant data differently and also data protection aspects are often difficult to ensure without appropriate system support. These aspects make interdisciplinary cooperation difficult, especially in the field of medical assessment.

Due to the digitization that has been going on for several years, an approach in the field of imaging and management of the images has been established. In the medical field, a picture archiving and communication system (PACS) is used. This system is an image archiving and communication system based on networking computing devices. The PACS captures digital image data from all modalities in radiology and nuclear medicine. In principle, images from other imaging modalities, e.g., endoscopy, cardiology, pathology, and microbiology can also be connected to the PACS system.

PACS systems provide the functionality of storing so-called "Key images" or "Presentation Status", in which the radiologist marks and saves a finding identified by him in the variety of stored examination images in a standard-compliant manner according to the standard "DICOM" and communicates it to other medical experts as an illustrated result of a radiological report (via PACS, or partially displayed in the report). The standard "DICOM" is a communication standard for connecting PACS-server with imaging devices regardless of manufacturer.

However, this approach has the disadvantage that the user sees exactly only the previously defined view of the findings and an "own" analysis (e.g., views of the neighboring tissue structures) is impossible. An advancement of this approach is to use bookmarks for the representation of 2D or 3D image data (3D Presentation States) that have been implemented proprietary in dedicated advanced visualization, AV, systems. But this still has the disadvantage that only experts of the same discipline (e.g., radiology) and only those who have access to the special systems and are experienced in their operation can use such advanced viewing options.

A consequence of the lack of technical support (access to digital data of all disciplines and suitable visualization and communication techniques) is that communication between the disciplines is very limited or, in the established clinical case conferences in which the experts of various disciplines meet in person, only takes place in analog form using written notes on paper.

Due to lack of or unreliable access even to medically important information, critical decisions are often postponed or made under hypothesis with potential negative impact on the patient and health economics if, for instance, an examination have to be repeated. Since there is great time pressure during the examination, it is impossible to wait until each physician opens his/her own system and/or each physician starts searching in data in a system that is unknown to him/her.

Further consequences of the lack of technical support for old cases are that often only data of one's own discipline (e.g., radiology or pathology) are known and thus no complete health state representation of the patient can be conveyed. In addition, quality management of data is very costly because no standardized data sets with common and standardized annotations for larger data sets, e.g., datasets for all lung cancer patients in a year, are available. Moreover, a case collection with reference cases cannot be shared among each other because the stored information is not standardized and often incomplete.

Accordingly, it is an object of the present invention to at least partially overcome the disadvantages known in the prior art and providing a solution that allows an interdisciplinary cooperation having all data and information from all parties involved editable available for each party involved in the interdisciplinary cooperation.

This object is achieved with the subject matter according to the independent claims with the claimed features. Advantageous embodiments are the subject matter of the dependent claims, the description, and the figures.

According to a first aspect, the present invention refers to a method for digital cooperation for a group of collaborating computing devices by providing access to a health-related application persisting an application state for a medical assessment. The method comprises several steps.

In a first step a health-related application instance is executed on a sending computing device. The sending computing device provides means for accessing and controlling the executed health-related application.

In a further step, an application state of an executed health-related application instance is bookmarked on the sending computing device to be output and shared within the group of collaborating computing devices.

In a further step, the bookmarked application state of an application instance is stored by a backend computing device. The application instance is executed on the sending computing device. Further, by the backend computing device on the basis of the stored bookmarked application state a container as a data structure including health-related application data is generated.

Further, by the backend computing device a link referring to the generated container is generated.

In a further step it is foreseen that by the backend computing device or by the sending computing device a sending of the generated link to at least one receiving computing device of the collaborating computing devices is initiated.

Further, the link is received by the at least one receiving computing devices. Further, the received link is activated by the at least one further selected computing device for providing access to the health-related application data and thereby processing an instance of the container.

Further, by the backend computing device any processed instance of the container is stored and is make available for further collaboration within the group of collaborating devices.

In the following the terms used within this application are defined in more detail.

In connection with the present invention, the term "medical assessment" can be understood as a set of device-related examinations run on a human or animal being to determine their overall medical condition, their general health state, or to determine specific health related issues. The medical assessment may be performed e.g., by imaging devices generating images of the body or parts of the body, which can be improved, processed, analyzed by a machine and/or physician and/or user being an expert of the imaging devices.

In connection with the present invention, the term "computing device" can be understood as any electronic equipment controlled by a central processing unit, including desktop and laptop computer, smartphones, smartwatches, virtual reality headset, and tablets. It refers to a general-purpose device that can accept software applications for many purposes in contrast with a dedicated unit of equipment such as a network communication interfaces, communication interfaces, user interfaces, memory, storage, CPU, etc. The communication interfaces as well as the user interface are configured to receive control instructions for controlling the computing device. The computing device is a device connectable to a communication network and configured to communicate via the communication network to further computing devices and thus can be designed as a receiver as well as a sender of data, e.g., health-related application data, controlling data, authentication data, etc.

In connection with the present invention, a "group of collaborating computing devices" can be understood to include computing devices using tools, applications, and/or hardware to better facilitate group work between computing devices working at different locations, e.g., in-office, at different sites (multi-site), and remote from e.g., at home. A group can be further understood to be a private or public communication network where computing device having permission for accessing the group to be allowed to communicate to each other. The group of collaborating computing devices may comprise a sending device (for the person who wants to share data and/or functionality with Colleagues), and a receiving device (the recipient of such sending.

In connection with the present invention, the term "backend computing device" can be understood as a computing device being part of a system for separation data presentation (frontend) and data access, execution and/or administration (backend). The backend computing device may include a server for running a health-related application as well as instances of the health-related application. Thus, the backend computing device may be the backend in the group of collaborating computing devices configured for storing, administration, authentication, controlling, and/or communication purposes. Further, the backend computing device may be configured to provide all data requested by the frontend. Further, the computing device is configured to output the bookmarked health-related application state. Outputting the bookmarked health-related application state includes displaying the information related to the bookmark on a screen, e.g., a monitor, liquid-crystal display, etc. storing the information related to the bookmark for later (requested) displaying, or providing all information to be output by a voice information signal via a speaker.

In connection with the present invention, the term "digital cooperation" can be understood as the purposeful interaction of two or more systems, in particular two or more computing devices. Cooperation may be based on having common goals. The two or more systems may communicate and interact with each other and share data and/or applications for execution. In particular, the two or more systems may have different functionalities and/or resources through which they support and complement each other.

In connection with the present invention, the term "health-related application" can be understood as a program to be executed on a computing device, which can be used, in order to work on or support a useful or desired health-related task, functionality, and/or diagnoses. A health-related application includes a sequence of instructions (consisting of declarations and instructions) satisfying the rules of a certain programming language in order to process or solve certain functions or tasks or problems with the aid of a computing device. A health-related application does nothing as long as it has not been started. If a health-related application is started - more exactly: a copy (in the main memory) of the health-related application (on the read-only memory), then this instance becomes an (operating system) instance, which must be assigned to a processor core to run.

In connection with the present invention, the term "health-related application instance" can be understood as a health-related application at runtime. More precisely, a health-related application instance is a concrete instantiation of a health-related application for its execution within a computing device, supplemented by further (management) information and resource allocations of the operating system for this execution. A health-related application instance is further the runtime environment for a program on a computing device, as well as the binary code of the program embedded in during execution. A health-related application instance is dynamically controlled by the operating system by certain actions, with which the operating system sets it into appropriate conditions.

In connection with the present invention, the term "application state" can be understood as the state at which a health-related application instance resides. An application state may indicate to where and what portions in the health-related application is/are being executed and the memory that is stored for the health-related application instance. In particular, from the application state a snapshot can be generated. A snapshot of the health-related application instance is a particular moment of the executed health-related application instance. All information from the health-related application instance in this particular moment are added to the bookmark, like e.g. variable settings etc. In this context, the bookmark includes information describing the application state in a particular moment.

More particular, the term "bookmark" can be understood as a saved shortcut that directs a computing device of a user (the creator of the bookmark of another recipient) to a certain set of information (e.g., portions) presented in a way to easily grasp the respective content. It may be a simple note, pointing to a certain location of interest in a medical image or a comprehensive arrangement of multiple sources of information putting together relevant context from a specific application state to be reviewed later on in context of a user. A user, for instance, a patient, another expert, or another person, having access rights to access the information. A bookmark usually references a certain location of interest (e.g., image/series number or a spatial location within a certain patient imaging exam) and stores links or copies of relevant context information, optionally together with visualization/information arrangement information from a specific application state. A bookmark contains all the necessary information to re-visualize the information for a reader reviewing the bookmark later on.

In connection with the present invention, the term "container" can be understood as a virtualization technique that separates applications including their runtime environments. A container does not contain their own operation system. A container uses the very operation system of the system (computing device) on which they are installed and/or executed. A container has a defined structured format to store data and/or applications. An application can be a software application to be executed on a computing device. A container may virtualize applications. The applications are strictly separated from each other, although they run on the same computing device and/or operation system. The container does not emulate their own operation system, but uses the operation system of the host system. A host system is the computing device to open the container and executing the content of the container. All data open in an applications instance, the state of the application instance, files, configurations, dependencies, annotations and/or libraries required for execution are present in the container. A delimited package with the runtime environment of the respective application can be created. The format of a container can be defined and easily transferred to other client and/or host systems. By means of the container, the user can move applications, in particular a health-related application instances between different system environments with minimal effort. All information and content are transported within one file. The container includes the set (content or links to selected content) of information selected (portion) to be presented when accessing a bookmark and the arrangement of such information. The container may be generated automatically by executing a corresponding script and triggered by specific events, e.g., when a bookmark to an application state is generated and/or stored and/or requested. The container may be stored and managed by the backend computing device hosting services to access bookmarks, e.g., by using a web-callup. The container may be construed as a compressed form of the presentation state.

In connection with the present invention, the term "link" can be understood, a reference to an object at another location, e.g., computing device, similar to a cross-reference. The object may include files, directories, containers, and/or software applications. The object may be stored at different locations including different computing devices, databases, memory, or are also managed under different names. Further, a link may also establish a relationship between two or more objects. A link can be provided and/or send and/or exchanged by using different communication mediums, e.g., email, text messengers, like in particular a chat program, data storages etc. A link can be exchanged between different computing devices in the group of collaboration computing devices as well as with computing devices included in a group of computing devices having permission for using and executing the link.

In connection with the present invention, the term "received and activated link" can be understood as the link that has been received by a receiving computing device in the group of collaborating computing devices or by a computing device having permission to receive and access the link. An activated link is a link that has to be activated by the providing device before the receiving device can execute the received link. An activation comprises providing a permission by the providing device to execute the link by the receiving device.

According to a second aspect, the present invention refers to a method for digital cooperation for a group of collaborating computing devices by providing access to a health-related application persisting an application state for a medical assessment. The method comprises several steps.

In a first step, a health-related application instance is executed by a sending computing device. The health-related application instance provides means for executing and/or accessing and/or controlling the executed health-related application.

In a further step, by the sending computing device an application state of the accessed health-related application on the sending computing device to be output and shared within the group of collaborating computing devices is bookmarked.

A further step comprises storing the bookmarked application state of an application instance by the backend computing device. The application instance is executed on the sending computing device.

In a further step, on the basis of the stored bookmarked application state, a container as a data structure including health-related application data is generated by the backend computing device.

A further step comprises generating a link referring to the generated container by the backend computing device. Further, a step initiating a sending is provided by the sending computing device or by the backend computing device. A sending of the generated link to at least one receiving computing device of the collaborating computing devices is initiated.

In a further step access for at least one receiving computing device to a health-related application configured for data processing of the application state in the container and thereby processing an instance of the container is provided by the sent link.

According to a third aspect, the present invention refers to a method for digital cooperation for a group of collaborating computing devices by providing access to a health-related application persisting an application state for a medical assessment. The method comprises several method steps.

In a first step, by at least one further selected receiving computing device a link referring to a container as a data structure including health-related application data is received.

In a further step, the container is accessed by the at least one receiving computing device by processing the received link.

In a further step, access to the health-related application, configured for data processing of the application state in the container is received by the at least one receiving computing device and thereby processing an instance of the container.

According to a fourth aspect, the present invention refers to a method for digital cooperation for a group of collaborating computing devices by providing access to a health-related application, persisting an application state for a medical assessment. The method comprises several method steps, which are executed on a backend computing device.

In a first step, bookmarking signals from a sending computing device are received. The bookmarking signals indicate a bookmarked application state of an application instance of the accessed or executed health-related application. The application instance is executed or accessed on a sending computing device.

The bookmarking signals are for bookmarking an application state on the sending computing device to be output and shared within the group of collaborating computing devices.

Further, the method comprises the step for storing the bookmarked application state of the application instance. The application instance is executed on the sending computing device.

In a further step, a container as a data structure including health-related application data is generated on the basis of the stored bookmarked application state. Further, a link referring to the generated container is generated. The generated link is provided to the sending computing device (for transmission to the receiving computing device) or directly to the receiving computing device in a further step. Further, access for at least one receiving computing device to the health-related application for data processing of the application state in the container is provided and thereby generating a processed instance of the container.

The methods and systems according to this invention have several advantages. The present invention provides a common infrastructure and user interface usable by different medical disciplines that connects all required information sources from the different medical disciplines for a common usage. By the method and system an aggregation of relevant data can be created more efficiently and can be directly communicated to devices in medical disciplines which require all available data for a medical diagnoses/treatment by using only one single system. The aggregation provides a comprehensive view of data, which in general are not accessible and manageable by each medical discipline itself. By the present invention, both, an asynchronous communication as well as a synchronous communication is supported.

The synchronous communication may relate to working on the file by different computing devices (and users) in parallel or in the same moment. Synchronous communication may relate to a real-time communication between two computing devices, like via chat or messenger functions. In this context, the synchronous communication may advantageously be relevant for the appraisal of pre-prepared views in medical issues. The pre-prepared view allows different medical disciplines a common assessment of the medical issue.

The asynchronous communication may relate to using an asynchronous communication protocol. The asynchronous communication may relate to any type of communication that includes a time lag between when the device imparting the information sends the message, and when the device receiving the message interprets it. In this context, the asynchronous communication may be advantageously be relevant for an elaboration of decision papers/medical findings on the basis of the information provided by the different medical disciplines.

In addition, the procurement, provision, and/or the acquisition of medically relevant information according to the invention increases the quality of medical decisions, resulting in faster diagnostics and patient treatment.

Furthermore, the present invention can compress the medically available amount of data of the different medical data in such a way that only data necessary for the medical case, however in the necessary detail depth of all discipline-relevant information, are made available. A previous selection, transfer or conversion into the "own" system data structure (own here relates to the data structure of the recipient) for use is not necessary and thus reduces the evaluation time.

Furthermore, the patient safety is increased by the availability of case collections. Available cases can be added to collection for an improved and faster query. The case collections can be used for quality management. In addition, an improved and more intensive communication between different disciplines and a resulting feedback from the disciplines facilitates the access to comparable cases in the own case collection.

In an embodiment of the invention, the method further comprises at least the step of authenticating the collaborating computing device. In particular, the step comprises authenticating the receiving computing device. In a further step, the method comprises providing access for the authenticated receiving computing devices, in particular to the container by activating the received link. The authentication is used to determine whether a computing device, in particular the receiving computing device or a user using the receiving computing device is actually the device/user who it/he/she claims to be and is allowed to access the data. An authentication typically consists of several steps, which can be differentiated in providing a specific identifier, verification of the specific identifier, and authorization when the verification was successful. Using an authentication process prevents access to medical and personal data by devices/user which/who are not authorized.

For authentication of the involved computing devices methods based on different identifiers can be used. For example, in some authentication methods no physical object is required to perform the authentication. For the login process on the computing device, it would be necessary to input e.g. the user's name and the corresponding password as credentials. The advantage is that these solutions are usually very easy to implement and to use, take less time to implement, and are also very inexpensive.

Other authentication methods are based on a tangible object such as a key, an ID card or an RFID card for authentication. The advantage here is also the ease of use. In addition, the security standard is higher than with the methods based on knowledge.

Other authentication methods are based on biometrics (physical characteristics) by authenticating by means of physiology-based or behavior-based characteristics. With physiology-based characteristics, permanent, external features that do not change are used for authentication. For example, facial recognition, iris recognition or fingerprints are used here. Behavior-based characteristics are active actions performed by a person. Examples include voice recognition or signature scanners.

Advantages of said methods are that they are very secure and yet remain quite simple in their operation. In order to meet the highest safety standards, there is the possibility to combine the different methods.

In a further embodiment of the invention generating by the backend computing device includes that the link referring to the generated container is executed after receiving a request signal issued by the sending computing device. The link is generated and available when requested by the sending device. This ensures, that the concrete application state of the application instance to be shared to other collaborating computing devices is achieved and available for sharing. An unauthorized access is impossible.

In a further embodiment of the invention the bookmarking of an application state comprises generating a bookmark pointing to the application instance of the bookmarked application state by using the means provided with the sending computing device. In particular, the specific bookmarked application state of the application instance process at the sending device includes all image series activated or selected, open documents, as well as notes and annotations added to image series. The specific bookmarked application state is stored by a trigger event generated on the sending device. The sending device may include a user interface to interact and control the sending device.

In a further embodiment of the invention, the health-related application data comprises application features of the executed health-related application instance. Application features are tools delivered by the executed health-related application instance that can be used within the application instance to complete a set of tasks or actions to perform e.g., a diagnosis. For instance, the health-related application instance provides features to open a series of images made with an imaging modality, like an MRI scanner. These opened images can be turned, moved, it can be zoomed in and out, etc. The application features support a user or physician to handle the image set made and to analyze the images. Further, application features also include the data/image handling including storing and sharing. It has to be noted, that all functionalities of the application instance which is executed on the sending computing device are accessible and possible to be executed on the receiving computing device as well, irrespective of whether the application is also loaded in a memory of the processing unit of the receiving node. For example, if a radiologist works with a very extensive viewing application, providing a huge set of functions and may be a proprietary software application, all of the provided functionality (of the radiologist) is transferred to the receiving computing device and may be used there, irrespective of the installation on the receiving computing device, which may e.g. be a patient's device or may be a clinical colleague from another discipline who has not installed the extensive radiologist's applications. This is possible, by providing the link with the container.

In a further embodiment of the invention, the health-related application data comprises the application state, enriched with meta information. Advantageously, an application state of a health-related application instance includes all attached and opened as well as modified images of an image set that a user using a computing device have been working on. This application state can be stored and provided to further computing devices as well as shared by using different communication mediums, e.g., email, chat programs, etc. Further, the user or the computing device can enrich the application state with meta information that a receiving computing device or a user using the receiving computing device can use to work with the application state of a health-related application instance.

In a further embodiment of the invention, the health-related application data comprises accessed and bookmarked health-related data. Advantageously, the health-related application data refer to specific data (bookmarked health-related data) selected and made useable for a medical diagnose and can be shared with further computing devices.

In a further embodiment of the invention, the health-related application data comprises references to literature, in particular scientific literature. Advantageously, the health-related application data referring to literature that can be shared using the bookmarked application state.

In a further embodiment of the invention, the health-related application data comprises medical knowledge associated to the case to be collaborated on in particular in an encoded form. The medical knowledge associated to the case can be used for the medical diagnoses and can be shared by using the bookmarked application state to obtain a second opinion/diagnoses. Further, the option to obtain a second opinion is provided. Thus, only user involved in this case having appropriate access rights are allowed to access the case. An authentication can be used to verify the corresponding access rights.

In a further embodiment of the invention, the health-related application data comprises chat data, in particular chat history data. Using chat data, communication connections between two or more computing devices can be re-established. Further, the chat history data can be used to track changes that have been made to the application data or the health-related application instance. Further, it can be tracked, which user was involved in chat.

In a further embodiment of the invention, the health-related application data comprises a unique identifier associated to the generated container. The unique identifier can be used to identify the corresponding container including the required data for sharing and editing the health-related application instance.

In a further embodiment of the invention, the link is generated according to a communication system for processing the link and includes at least a reference to an allocated memory storing the container. The container is stored in a memory on a backend computing device, in particular a plurality of container and variants of the container are stored on the backend. The link can include a uniform resource locator (URL) pointing to a memory storing the container. A part of the URL can be the reference. The URL support an identification of the stored or requested container as well as a further processing.

In a further embodiment of the invention, the container is a result of a database query.

In a further embodiment, the data represented in the container is dynamic so that the health-related data, represented in an instance of the container may differ from container to container and even from instance to instance of the same container. Using the container, content of a health-related application instance and stored to a specific moment (application state), can be queried from different sources, including different discipline databases and streamed to another participating computing device. Further, the streamed health-related application instance can be amended by adding further documents, information, annotations, etc., which result in new instance of the container. This new instance can be further shared or provided to other collaborating computing devices.

In a further embodiment of the invention, executing a health-related application instance comprises aggregating data from different heterogeneous data sources and linking the aggregated data to the container using the meta information and/or executing related additional applications, accessible via the health-related application. Under different heterogeneous data sources can be understood sources that provide data that support or can be used by a user or physician for his/her medical assessment of a patient. The different heterogeneous data sources are connected over via a communication network and provide the data to be aggregated in a readable format. Advantageously, the aggregated data can supplement already existing information or replace it with further findings and thus make a diagnosis/treatment more accurate and efficient.

In a further embodiment of the invention, the collaboration application provides means for exchanging reference cases for the collaboration case. Advantageously, results achieved within a medical assessment can be exchanged as reference case within the group of collaborating computing devices. Further, even a case has been opened/started, the case can be exchanged can be exchanged within the group of collaborating computing devices for receiving support within the assessment.

In a further embodiment of the invention, the collaboration application provides means for annotating. Annotations provide further explanations or notes for specific scenarios. A user can use means of the computing device to add additional information to the images opened that a further user can read and use for his/her own analysis. Further, the annotations added can be amended by adding further information. Further, annotations record things that are not considered essential to the image, but are important additional information.

In a further embodiment of the invention, the collaboration application provides processing the health-related application. By the backend computing device, a health-related application can be provided (streamed) to another computing device of the group of collaborating computing devices as an instance of the health-related application. The health-related application is processed and only a stream is provided to the receiving computing device that needs less CPU performance and memory to output the instance of the health-related application.

In a further embodiment of the invention, the collaboration application provides data of the health-related application. A computing device and the user/physician retrieve the data receiving an instance of the health-related application. The data is provided and can be used for assessment as well as amended by adding further details, sources, and/or annotations.

In a further embodiment of the invention, the annotations are structured in a standardized format, like highlighted regions, overlays, tags, comments, disease classifications, key images etc. Structured annotations support the analytical analysis of the images and make the diagnoses/treatment of a patient quicker and more efficient.

In a further embodiment of the invention, the method is operated in a synchronous mode. This allows a direct communication between the sending computing device and the receiving computing device.

In a further embodiment of the invention, a sending computing device, which has requested the link and a receiving computing device, which has received the link are positioned in different software and hardware environments with different viewing applications. Advantageously it is possible that medical experts work together located at different cites und using different hardware. In particular, the backend computing device provides an instance of the health-related application via the received link that can be opened and viewed independently of the software and hardware available.

In a further embodiment of the invention, sending the link may be done via a chat function. A chat function is a real-time communication medium for networking with other computing devices in a single chat area or in a group chat area. The chat function is used to provide the receiving computing device the link to the container for opening and starting the health-related application instance. Also, the receiving computing device may resend a link using the chat function pointing to an amended container. Further, the chat function can be used to send text messages to connected computing devices. Further, the chat function can be used to send portions of the container as a context information in a multimedia message including a small thumbnail with a description. The chat function may also include functionalities for inviting further collaborating computing devices, get a note when a connected computing device is writing or sending data/information, and/or get a note when a connected computing device received and read a message sent.

In a further embodiment of the invention, the health-related application is based on a proprietary software. A proprietary is a non-free software, where a license has to be acquired before using the software. According to the acquired license, the software can be installed on a single system or on multiple systems. Further, according to the license, the scope of functionality can be used fully or is limited according to a license plan. Different license plans offering different sets of functionality scope can be provided. The proprietary software is a software that e.g. can be used to analyze the set of images made with an image scanner, e.g., MRI-scanner. The proprietary software may be executed on a sending computing device or on a backend computing device and streamed to a requesting computing device (frontend). In particular, an image stream is provided including the images of the bookmarked application state combined with further information. For the image stream no further program software is required to display the images on a receiving computing device.

Up to now, the invention has been described with respect to the claimed methods. Features, advantages or alternative embodiments mentioned with respect to the method can also be assigned, transferred or, applied to the other claimed or described subject matters (e.g., system/apparatus the computer program or a computer program product) and vice versa. In other words, the subject matter of the apparatus/system can be improved or further developed with features described or claimed in the context of the method and vice versa. In this case, the functional features of the method are embodied by structural units of the system, configured to dedicatedly execute this function and vice versa, respectively. Generally, in computer science at least from a computational point of view, a software implementation and a corresponding hardware implementation are equivalent, at least from a computability perspective. Thus, for example, a method step for "storing" data may be performed with a "storage unit" and respective instructions to write data into the storage. For avoiding redundancy, these embodiments are not reiterated or explicitly described again for the apparatus, because they have been described already in relation to the method.

According to a fifth aspect, the present invention refers to system for digital cooperation for a group of collaborating computing devices by providing access to health-related application persisting an application state for a medical assessment.

The system comprises a set of collaborating computing devices. Each collaborating computing device comprises a processing unit, a user interface, and a communication interface. At least one collaborating computing device of the set of collaborating computing devices acts as a sending computing device and at least another one collaborating computing device of the set of collaborating computing devices acts as a receiving computing device and still another computing device acts as a backend computing device.

The system further comprises distributed storages for storing a health-related application and in particular different instances of the health-related application. The different instance of the health-related applications may be executed by the processing unit of the computing devices. The user interface of the sending computing device is configured for selecting an application state of the executed health-related application instance.

Further, the computing device acting as the sending computing device is configured for executing, accessing and/or controlling a health-related application instance. Said sending computing device is further configured for bookmarking an application state of the executed health-related application instance. The application state is to be output and shared within the group of collaborating computing devices. Said sending computing device is further configured for initiating a sending of a generated link to at least one further selected computing device of the collaborating computing devices for activating the sent link by the at least one receiving computing device for providing access to the health-related application data and thereby processing an instance of the container.

Further, the computing device acting as a backend computing device is configured for storing the bookmarked application state of an application instance, being executed on the sending computing device. The computing device acting as a backend computing device is further configured for generating on the basis of the stored bookmarked application state a container as a data structure including health-related application data. Further, said computing device is configured for generating the link referring to the generated container, and storing any processed instance of the container and making it available for further collaboration within the group of collaborating devices.

In an embodiment of the fifth aspect of the invention, the system is further configured for authenticating the computing devices and in particular authenticating the receiving computing device. The system may further be configured for providing access for the authenticated computing devices to the container by activating the received link.

The invention includes a communication system comprising at least three different layers providing interfaces for communication and data transfer between the different parties of the system.

In an embodiment of the fifth aspect of the invention, the system comprises at least an information access layer (IAL).

The information access layer is configured to be the bridging part between a user interface and the several information providing systems that are storing and providing specific medical health-related information e.g., DICOM image data, structured report (analysis for a medical diagnoses/treatment) in PACS, unstructured physician's letter in a LIS-system (laboratory information system), and/or clinical information in a HIS-system (hospital information system). Thus, the information access layer provides/includes interfaces for communication with different data sources and to support the data transfer.

DICOM (Digital Imaging and Communications in Medicine) is the standard for the communication and management of medical imaging information and related data. DICOM is used for storing and transmitting medical images enabling the integration of medical imaging devices such as scanners, servers, workstations, printers, network hardware, and picture archiving and communication systems (PACS) from multiple physicians. The laboratory information system (LIS) is a software-based application for managing data and supporting workflows in laboratories that are sample-oriented such as analytical laboratories. LIS support sample processing and related workflows. They provide transparent tracking of samples throughout the entire sample processing cycle in the laboratory, make laboratory operations efficient, and ensure appropriate quality management in a regulated environment. HIS (Hospital information system) is the class of the totality of all information processing systems of information technology for the collection, processing, and transfer of medical and administrative data in the hospital. HIS includes server functions, workstation functions and mobile data provision functions. HIS may also include conventional methods of paper-based documentation and voice communication.

Further, the information access layer (IAL) provides functionalities for administration of user specific authentication methods of different systems. For authentication can be used a password authentication, email related authentication, short message service (SMS), applications running on a separate device (app), physical aids including hardware token, biometric features, two-factor authentication and/or single-sign-on.

By using a password query, a checksum (hash) of the password is calculated in the browser after it is entered and compared with a hash value stored on the server during registration. This is a method from the knowledge category. This method can therefore be used without the need for additional tools such as a cell phone or smart card. It is essential for security that the password is kept secret and cannot be read or calculated by third parties. The stored hash on the server can be further secured, for example by using a salt value that is included in the calculation of the hash value. This, in conjunction with the use of a sufficiently strong hash function increases security.

Authentication can also be performed by sending an e-mail. In this case, the process is started on the respective website, whereby an e-mail is sent to the e-mail address stored for this computing device/account used to login into the computing device. This mail contains either a link that leads directly to the account or a one-time password (OTP) that must be entered in the login mask. This proves ownership of the email account or email address, which in turn is secured with access authentication.

Using the short message service, a message is sent to a register mobile phone number specified during the registration process. The message received on the mobile phone contains a code that must be entered on the website. This proves the ownership of the cell phone registered under this number or the SIM card inside it. Similar to the short message service method, an application (APP) can also be installed on a smartphone, which, when activated, provides a code to enter on the website or sends a positive feedback to the web service through other confirmation.

Authentication including hardware tokens may comprise reading information from USB sticks, smart cards, or similar hardware that are delivered to a specific computing device or user of the computing device and assigned to that computing device/user. Such tokens then either have a display on which regularly changing random numbers or passwords are shown, which then have to be entered when logging in. Others do not have a display (e.g., smart cards) but are connected to the computer and send the generated value or a signature created with a private key on it to the program via software interface. All variants are used to prove ownership of the assigned token.

A biometric feature can be used in combination with a smartphone. A voice recognition can be performed or fingerprint, iris or face features are scanned via an included camera for authentication. The characteristics identified cannot be changed or passed on, and a corresponding recording device is required for the respective capture of the biometric feature.

Using a two-factor authentication increases the security level, as a combination of the before mentioned factors are required before a valid authentication is established. A combination of two factors from different categories is used for this purpose. For instance, a password (knowledge category) is required before an SMS including a code is sent to the stored mobile phone number (possession category).

Using the single sign-on authentication, a service (e.g., Google, Facebook or Apple) confirms another page on which the user wants to log in that the person has the credentials to login.

Further, the information access layer (IAL) normalizes the read information from the several data source to a general patient data model for further usage by the involved physicians in the medical assessment (for initiating further examinations, diagnoses and/or treatment). The normalization can be performed automatically according to before set parameter settings choses individually by the physician/viewer.

Further, the information access layer (IAL) provides an interactive user interface for accessing or query the patient information. The interactive user interface that is configured to display communicated aggregated information content in a way that is appropriate for the receiving computing device. The information access layer allows the information to be viewed in various depths of detail. Further, the information layer allows additional content that is not primarily prepared to be viewed and to be communicated back as a feedback to any question the communication partner may have, together with textual or verbal descriptions. The user interface can be used on various devices (e.g., work station, computer, laptop, smartphone, tablet, smartwatch, beamer, etc.) and allows both synchronous and asynchronous communication.

In a further embodiment of the fifth aspect of the invention, the system comprises at least an information model layer. The information model layer receives the output of the information access layer. Different information or information fragments (specific document, values from the document, etc.) are combined into a semantic state, in particular in an application state automatically by using different wizards or by user interaction including a manual selection. The processes executed are represented as event chains. An event chain is listing of events. An event can be related to other events, which will create event chains. These event chains can significantly affect the course of the processes. Events can be correlated with each other without one triggering another one. The fragments of the event chain are represented in the cache. This result in a faster and more efficient access to the cache memory, when new and similar event chains are triggered.

The information model layer is used to map time-critical information, persistent information, and/or process results in the cache. A specific view including a processed instance is stored and provided by using the type of data as used, the semantic link between the different types of data as well as the knowledge about the user's field of expertise and the meaning of the data. The output of the information model layer is a semantic state of executed health-related application instance. The semantic state my include open files, data specific states, markings, notes, zooming information, etc.

In a further embodiment of the fifth aspect of the invention, the system comprises at least a communication layer. The communication layer (CL) is the layer used for bookmarking an application state of an executed health-related application instance. For generating the bookmarked health-related application instance is the output (result) of the information model layer including the semantic state. The communication layer has access to annotations/augmentations of the semantic state, which cannot be extracted from the patient data sources, in particular are no part of the sources and has been added later on in particular assessments. The semantic state and the annotations/augmentations are stored by the communication layer in specific databases, e.g., a bookmark database together with further meta information, e.g., creator of the data, date of creation, specific tag, etc. The database is a system for electronical data administration configured to store a large quantity of data efficiently, without contradiction, and permanently. The database may be used to bundle different bookmarks in containers with additional meta information. Each stored bookmark may be identified with a random generated but unique identifier. The identifier can be used for a synchronous communication including a chat inside a bookmark as well as for an asynchronous communication including loading a bookmark, changing the bookmark and thus creating a new, associated bookmark, and saving both bookmarks in a container.

Further, the communication provides the reference to the stored bookmark in the database that can be used to access the database for usage. The access for a receiving computing device is organized and managed by the information access layer. Using the reference, the sematic state can be loaded to the cache memory. Further, stored annotations/augmentations as well as meta information (creator, associated container) are loaded from the database and can be output for a receiving computing device.

In a further embodiment of the fifth aspect of the invention, the user interface is configured as a web interface. A web interface is an interface to a system that can be accessed via the hypertext transfer protocol (HTTP). The web interface is designed as a graphical user interface (GUI) through which a user can interact with the system by using a web browser or a web service through which the system provides data or functions to other systems. The web interface has the advantage to be platform-independent, since a web browser is available for almost every network compatible operation system of a computing device and will be supplied with the operation system, so that no additional software needs to be installed to use the web interface. The use of a web service for data exchange between two systems is advantageous, since data transport via HTTP is established independently of programming language and across platforms, and is usually also permitted through firewalls.

In a further embodiment, the system comprises an archiving interface to store specially prepared cases and make them available in collections, for instance, for clinical conference and teaching purposes.

According to a sixth aspect, the present invention refers to a computing device for digital cooperation for a group of collaborating devices by providing access to health-related application persisting an application state for a medical assessment. The computing device comprises a processing unit, a user interface, and a communication interface. The processing unit is configured for acting the computing device as a sending computing device or as a receiving computing device.

In case the computing device is configured as the sending computing device, the processing unit and the user interface are configured for accessing, executing and/or controlling a health-related application instance. Both are further configured for bookmarking an application state of the executed health-related application instance, wherein the application state is to be output and shared within the group of collaborating computing devices. Further configured for initiating sending a generated link to at least a further selected receiving computing device of the collaborating computing devices, and for providing by the sent link access for at least one further selected receiving computing device to a collaboration application configured for data processing of the application state in the container and thereby processing an instance of the container.

In case the computing device is configured as the receiving computing device, the processing unit and the user interface are configured for receiving a link to a container referring to a container as a data structure including health-related application data. Both are further configured for accessing the container by processing the received link, and for receiving access to a collaboration application configured for data processing of the application state in the container and thereby processing an instance of the container.

According to a sixth aspect, the present invention refers to a backend computing device for digital cooperation for a group of collaborating devices by providing access to health-related application persisting an application state for a medical assessment.

The backend computing device comprises a processing unit and a communication interface configured for interacting with a sending computing device and a receiving computing device. Said backend computing device further comprises distributed storages for storing different instances of a health-related application, which may be accessed by the processing unit. The processing unit is configured for receiving a bookmarked application state of an application instance, being executed on a sending computing device. The application state is to be output and shared within the group of collaborating computing devices. Said processing unit is further configured for storing a bookmarked application state of an application instance and generating on the basis of the stored bookmarked application state a container as a data structure including health-related application data. Further, said processing unit is further configured for generating a link referring to the generated container and providing the generated link to a receiving computing device. Further, the processing unit provides by the generated link access for at least one receiving computing device to the health-related application for data processing of the application state in the container and thereby generating a processed instance of the container.

According to a seventh aspect, a computer program is provided. The computer program is loadable into a memory unit of a computing unit. The computer program includes program code sections which induce the computing unit to carry out (execute) the steps of the method for digital cooperation according to the methods of the present invention and the aforementioned advantageous embodiments, when the computer program is loaded into a memory of the computing unit and executed in said computing unit. The realization of the invention by a computer program has the advantage that already existing processing units, such as computing devices (computer), handhelds, or server units can be easily adopted by updates in order to work as proposed by the invention.

It has to be pointed out that generally, features and/or embodiments, described so far may also be combined. Further, it is to be pointed out that software typically is modular in nature. Thus, a specific implemented feature which is mentioned in combination with a certain embodiment may also be combined with other features, even when mentioned in other embodiments. Accordingly, any feature may be combined with at least any other feature, which is claimed and/or described in this application, even when in another context.

The order, according to which the steps of the methods of the present invention are described in the present specification, does not necessarily reflect the chronological order, according to which said steps are carried out.

The properties, features and advantages of this invention described above, as well as the manner they are achieved, become clearer and more understandable in the light of the following description and embodiments, which will be described in more detail in the context of the drawings. This following description does not limit the invention on the contained embodiments. Same components or parts can be labeled with the same reference signs in different figures. In general, the figures are not for scale.

It shall be understood that a preferred embodiment of the present invention can also be any combination of the dependent claims or above embodiments with the respective independent claim.

These and other aspects of the invention will be apparent from and elucidated with reference to the embodiments described hereinafter.
Fig. 1 is a schematic representation of a system according to an embodiment of the invention;
Fig. 2 is a schematic representation of a computing device according to a further embodiment of the invention;
Fig. 3 is a schematic representation of a backend computing device according to a further embodiment of the invention;
Fig. 4 is a flowchart of a method for digital cooperation for a group of collaborating computing devices according to an embodiment of the invention;
Fig. 5 is a further flowchart of a method for digital cooperation for a group of collaborating computing devices according to a further embodiment of the invention;
Fig. 6 is a further flowchart of a method for digital cooperation for a group of collaborating computing devices according to a further embodiment of the invention;
Fig. 7 is a further flowchart of a method for digital cooperation for a group of collaborating computing devices according to a further embodiment of the invention;
Fig. 8 is a schematic representation of a time sequence for providing access to a health-related application according to an embodiment of the invention;
Fig. 9 is a further schematic representation of a time sequence for providing access to a health-related application according to a further embodiment of the invention;
Fig. 10 is a further schematic representation of a time sequence for providing access to a health-related application according to a further embodiment of the invention, and
Fig. 11 is a schematic representation of the communication system according to an embodiment of the invention.

Generally, the embodiments including features defining the method more precisely can be combined with features of the independent system claims as the components, in particular of the computing devices of the systems are configured to execute the steps of the method claims.

In the following possible embodiments of the different aspects of the present invention are described in more detail with reference to the enclosed figures.

The scope of the present invention is given by the claims and is not restricted by features discussed in the description or shown in the figures. Generally, any reference signs in the patent claims should not be construed as limiting the scope.

In the figures of the drawing, elements, features and components which are the same, have the same function and have the same effect - unless otherwise stated - are provided with the same reference symbols.

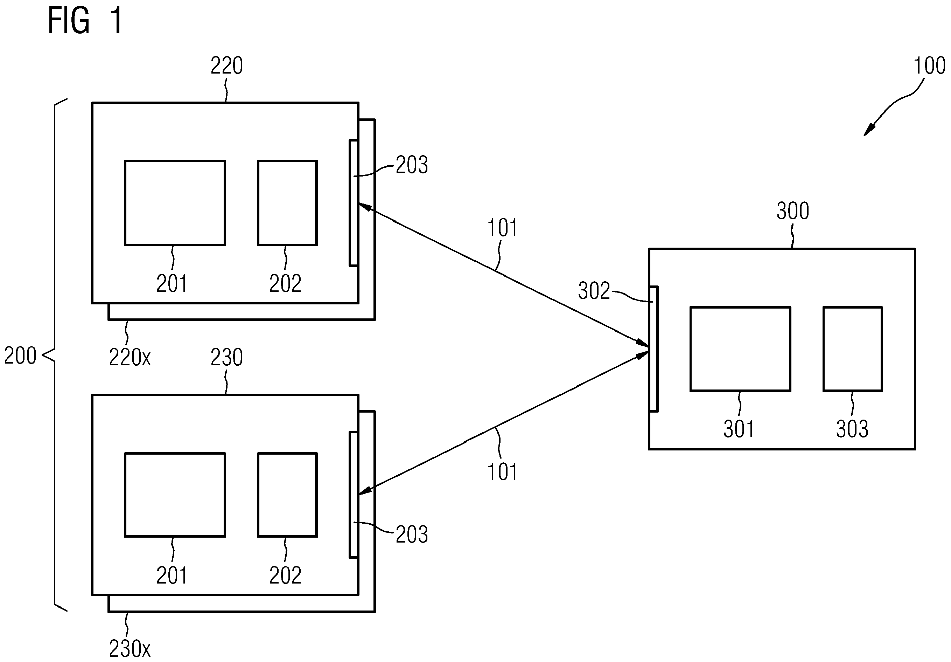

In the illustrated exemplary embodiment according to Fig. 1, a system 100 for digital cooperation for a group of collaborating computing devices 200 by providing access to a health-related application persisting an application state for medical assessment is shown. The system 100 in this exemplary embodiment is connected via a communication connection 101 (outlined by the arrows directing in both directions) to two exemplary computing devices 200. The computing devices 200 can be configured as a sending computing device 220 and a receiving computing device 230. The computing devices 220, 230 are connected to the backend computing device 300 via the communication connection 101. The communication connection 101 can be configured as a LAN connection using Ethernet or a WLAN connection using a WLAN access point. The computing devices 220, 230 as well as the backend computing device 300 can be either connected by a direct connection within a single network or by a connection using the Internet. Further, the invention is not limited to the exemplary embodiment as shown in Fig. 1. It is further possible that a plurality of computing devices 220x, 230x are included in the system 100 and connected via the communication connection 101 to the backend computing device 300.

In a further embodiment, the computing devices 220, 230 as well as the backend computing device 300 can be located at the same place as well as at multiple sites and communicating to each other using the internet and the communication connection 100.

In the illustrated exemplary embodiment, the system 100 comprises a set of computing devices 200. Each computing device 220, 230 of the set of computing devices 200 comprises a processing unit 201, a user interface 202, and a communication interface 203. The computing devices 200 can be configured as a workstation, a computer, a laptop, a smartphone, a tablet, or as a smartwatch. Each of the computing devices 200 comprises a communication interface configured to be connectable to the communication connection 101 for establishing a data transfer to the backend computing device 300 as well as from the backend computing device 300 to the connected computing devices 200. The processing unit 201 is configured to process the method according to the invention. The user interface 202 is configured to receive inputs from a user for controlling the computing device 200 as well as for receiving control commands for triggering the backend computing device 300. The user interface 202 is further configured to display the data included in the health-related application instance streamed by the backend computing device 300 to the computing device 200.

In the exemplary embodiment shown in Fig. 1, at least on computing device 200 of the set of computing devices acts as a sending computing device 220 and at least another one acts as a receiving computing device 230. The sending computing device 220 is defined as the device where a user provides an application state of a health-related application instance to be stored and shareable with other computing devices 200. The receiving computing device 230 is defined as the device where a user wants to receive a link to start a stream of the health-related application instance provided by the backend computing device 300 and shared by the sending computing device 220.

Further, the system 100 comprises distributed storages 303. The distributed storages 303 are configured for storing a health-related application. The distributed storages 303 are further configured to store different instance of the health-related application. The different instances of the health-related application are executed by the processing unit 201 of the computing devices 200. In the exemplary embodiment shown in Fig. 1, the distributed storage 303 is implemented in the backend computing device 300. The distributed storage 303 can also be provided by a further server (not shown) connected to the backend computing device 300 via the communication connection 101. The backend computing device 300 further comprises a communication connection to further computer/server of facilities including different medical tool/machines providing health-related data obtained within medical diagnoses, which can be combined with the health-related application for more in-depth diagnosis.

The user interface 202 of the computing device 200 is configured for selecting an application state of the executed health-related application instance. Selecting the application state in this context includes a selection of the images displayed, features active to display the images for a specific medical purpose, added notes for diagnosis or consultation of other experts, etc. These parameters can be stored.

A computing device 200 acting as the sending computing device 220 of the system 100 is configured for executing a health-related application instance. The health-related application may be executed on the backend computing device 300 and an instance is streamed to the sending computing device 200. The received stream of the health-related application instance is executed by a viewer processed by the processing unit 201 of the sending computing device 220. The viewer processed by the processing unit 201 is in communication with the backend computing device via a communication system for establishing a stream including the correct resolution possible to be displayed by the user terminal 202 of the sending computing device 220.

Alternatively, or in addition, the health-related application and in particular an instance thereof may be executed directly on the sending computing device 220. Then, the application state and the bookmark information are to be transferred from the sending computing device 220 to the backend computing device 300, for making the application state accessible for a receiving computing device 300.

Further, the user terminal 202 of the sending device 220 is configured to receive a control signal to bookmark an application state of the executed health-related application instance. The bookmarked application state of the current displayed and processed health-related application instance can be output and shared with the group of the collaborating computing devices 200 of the system 100 using the backend computing device 300.

Further, the user terminal 202 of the sending device 220 is configured to initiate a sending of a generated link to at least one receiving computing device 230. In a preparation step, the sending computing device 220 may issue a field and/or button on the user interface for selecting one or more receiving computing devices 220 from the set of collaborating computing devices. The receiving computing device is then configured and prepared for activating the sent link for providing access to the health-related application data and thereby processing the instance of the container. The sent link can also be activated by a plurality of receiving computing devices 230 included in the system 100 and connected to the backend computing device 300. The container includes all information required for streaming a health-related application instance to be processed on at least one (selected) receiving computing device. In a first setting, it is possible to select all collaborating computing devices 200 as potential receiving computing devices. In a second setting, only a subset of all collaborating computing devices 200 is set as potential receiving computing devices 230. In a third setting, only one computing device is selected as receiving computing devices 230.

The backend computing device 300 of the system 100 is configured to store the bookmarked application state of a health-related application instance processed by the processing unit 201 of the sending computing device 220. The bookmarked application state is used to generated a container with a specific data structure. The data structured can be based on the JavaScript Object Notation (JSON). JSON is a lightweight data-interchange format. The data structure is configured to store health-related application data. Further, the health-related application data may include meta-data referring to different sources of data required for providing the health-related application.

Further, the backend computing device 300 of the system 100 is configured to generate a link referring to the generated container. The link may include a URL link referring to a specific storage sector in the distributed storage 303 configured for storing a plurality of containers.

Further, the backend computing device 300 of the system 100 is further configured for storing any process instance of the container and making it available for further collaboration with the group of collaborating computing devices 200.

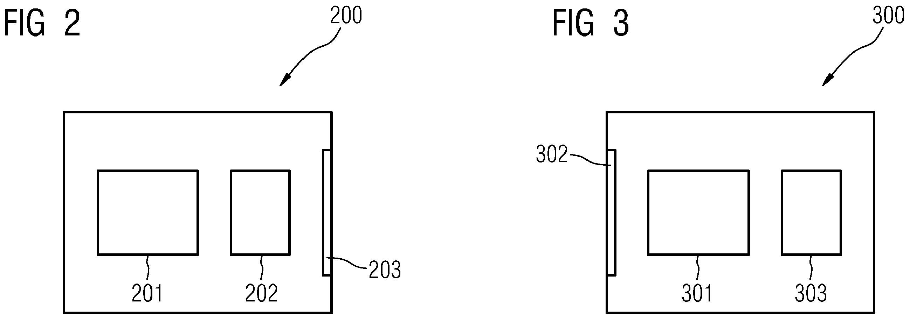

In the illustrated exemplary embodiment according to Fig. 2, a computing device 200 is shown. The computing device 200 is configured for digital cooperation for a group of collaborating devices by providing access to health-related applications persisting an application state for a medical assessment. The computing device 200 can be configured as a personal computer, laptop, workstation, a terminal connected to a server, a handheld, e.g., smartphone, tablet, PDA, or a smartwatch. Further electronical devices comprising at least a communication interface, a user interface, as well as a processing unit established to process a program software can be used in the sense of the invention. The computing device 200 can be configured as a sending computing device 220 (see Fig. 1) or as a receiving computing device 230 (see Fig. 1).

The computing device 200 includes several hardware components, e.g., a processing unit 201, a user interface 202, and a communication interface 203. The computing device 200 may also include several memories and storages as well as drives for data exchange on replaceable storage devices. The processing unit 201 is configured for acting the computing device 200 as a sending device 220 or as a receiving device 230. In this way, the processing unit 201 processes different part and/or steps of the method according to the invention.

The user interface 202 may comprise a display, screen or medium to display information to a user using the computing device 200. Further, the user interface 202 is configured to receive user control commands to control the computing device. The user interface 202 can be a touch screen, where a user provides control commands by touching specific areas on the touch screen. Further, the user interface 202 may include separate hardware for displaying information as well as controlling the computing device 200. A hardware for inputting control commands can be, for instance, a keyboard, a mouse, and/or voice- and face recognition.

The communication interface 203 is configured to establish a communication connection to a network, in particular connect the computing device via the communication connection 101 to the backend computing device 300 and/or to further computing devices 220x, 230 of the system 100. The communication interface 203 may include an LAN or WLAN interface.

In case the computing device 200 is configured as the sending computing device 220, the processing unit 201 and the user interface 202 of the sending computing device 220 are configured for executing and controlling a health-related application instance of a health-related application process by the backend computing device 230 and streamed to the sending computing device 220. Further, the sending computing device 220 is configured for bookmarking an application state of the executed health-related application instance. Thus, a user can create a snapshot of opened images, and may include the annotation, notes, open documents etc. on the sending computing device 220. This information specifying an application state is bookmarked. The application state is to be output and shared within the group of collaborating computing device 200. In particular, the application state can be stored at backend computing device 300 (see Fig.1) and shared.

The sending computing device 220 is further configured to initiate sending a generated link to at least one receiving computing device 230 of the collaborating computing devices 200. A link includes the reference to a container stored in the distributed storage 303 of the backend computing device 300 or a distributed storage 303 in communication connection with the backend computing device 300. The container includes all information to establish and stream an instance of the health-related application as requested. The link is a unique link pointing to the memory location storing the container. The link may include as unique link the reference of the container. The link can be sent via a communication medium including a chat program, email, short messages service, exchange on a hardware medium, etc.

The sending computing device 220 is further configured for providing access for at least one receiving computing device 230, 230x to a collaboration application configured for data processing of the application state in the container and thereby processing an instance of the container. This provision of access is possible and exeduted by the sent link. The selected receiving computing device 230, 230x is configured to receive the link and process the link by opening it in an application, e.g., a browser, web browser.

In case the computing device 200 is configured as the receiving computing device 230, the processing unit 201 and the user interface 202 are configured for receiving the link to the container referring to a container as a data structure including health-related application data. The data structure can be a JSON, XML, or further data structure structuring data in a readable manner. The received link is pointing to the container stored on the backend computing device 230 including the bookmark application state of a health-related application instance provided by the sending computing device 220. The received link can be processed by the processing unit 201 of the receiving computing device 230. By processing the received link, the container can be accessed. Further, the processing unit 201 and the user interface 202 are configured for receiving access to the collaboration application configured for data processing of the application state in the container and thereby processing an instance of the container. The instance of the health-related application is streamed by the backend computing device 300 to the receiving computing device 220 and processed by the processing unit 201 and displayed in a viewer application at the user interface 202.

In the sense of the invention, both, the sending computing device 220 as well as the receiving computing device 230 may comprise the same hardware configuration. Further, the sending computing device 220 and the receiving computing device 230 are configured to bookmark an application state of a health-related application instance and to share it, respectively to receive a link for a shared application state of a health-related application instance.

In the illustrated exemplary embodiment according to Fig. 3, a backend computing device 300 is shown. The backend computing device 300 comprises a processing unit 301 and a communication interface 302 configured for interacting with a sending computing device 220 and a receiving computing device 230. The communication interface 302 can be connected to the communication connection 101 (see Fig. 1) by using LAN and WLAN.

Further, the backend computing device 300 comprises distributed storages 303 for storing different instance of a health-related application. The different instances of the health-related application are accessed by the processing unit 301. The distributed storages 300 can be included in the backend computing device 300 or located on a different site (e.g., further server, external storage drive, etc.) and connected to the backend computing device 300. The distributed storages 303 may include a database or a database system that is a system for electronic data management. The essential task of the database is to store the containers efficiently, without contradiction and permanently, and to make the container available to the computing devices 200.

The processing unit 301 of the backend computing device 300 is configured for receiving a bookmarked application state of an application instance, being executed on a sending computing device 220. The application state is to be output and shared within the group of collaborating computing devices. The bookmarked application state of the application instance is stored in the distributed storage 303.

The processing unit 301 of the backend computing device 300 is further configured to generate on the basis of the stored bookmarked application state a container as a data structure including health-related application data. Alternatively, or in addition, the container may include functional data for the application, i.e. software routines and functionalities, which are to be provided to the receiving computing device 230.

The generated container can be provided to further receiving computing devices 230 having access to the container. The data structure may include a JSON or XML data structure.

Further, the processing unit 301 of the backend computing device 300 is configured to generate a link referring to the generated container stored in the distributed storage 303. The container includes a unique reference that is also included in the link pointing to the storage location storing the container. The link can be built as a URL. The generated link is provided by the backend computing device 300 triggered by the sending computing device 220 to the receiving computing device 230. A receiving computing device fulfilling all access credentials can open the received link.

Further, the processing unit 301 is configured to provide access by the generated link for at least one (selected) receiving computing device 230 to a collaboration application for data processing of the application state in the container and thereby generating a processed instance of the container on the receiving computing device 230.

The backend computing device 300 provides an image stream of the application state stored in the container when requested by a receiving computing device 230. The image stream may include a plurality of images that may be split on the backend computing device 300 side in several matrix parts. The backend computing device 300 may determine, when streaming the image stream, which matrix parts are changed and the matrix is transferred including only the changed matrix parts. Parts unchanged can be ignored and don't have to be transferred. The matrix parts are assembled at the receiving computing device 230 after the transfer is finished. This may lead to a more efficient usage of the bandwidth and increases the data transmission rate.

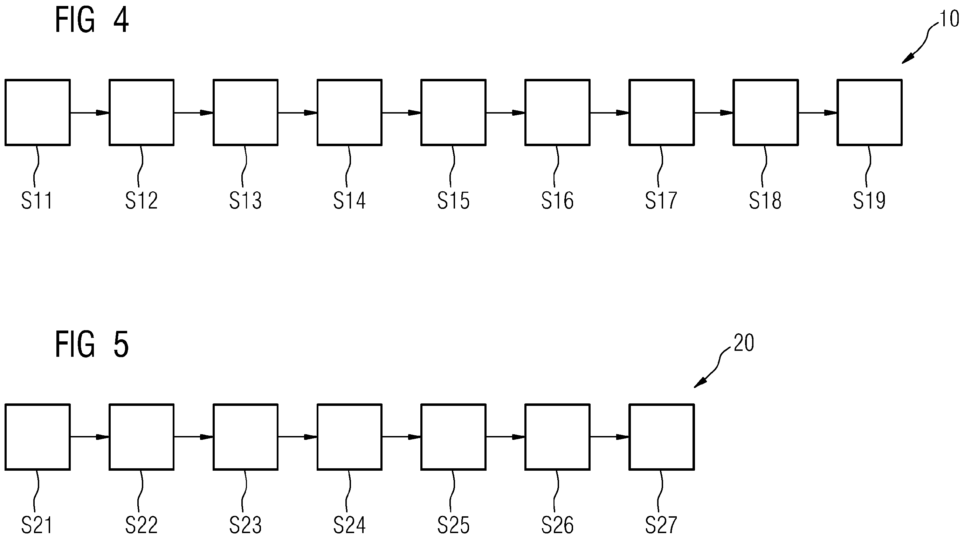

Fig. 4 shows a flowchart of a method 10 for digital cooperation for a group of collaborating computing devices according to an embodiment of the invention. In the illustrated exemplary embodiment according to Fig. 4, method 10 comprises several steps.

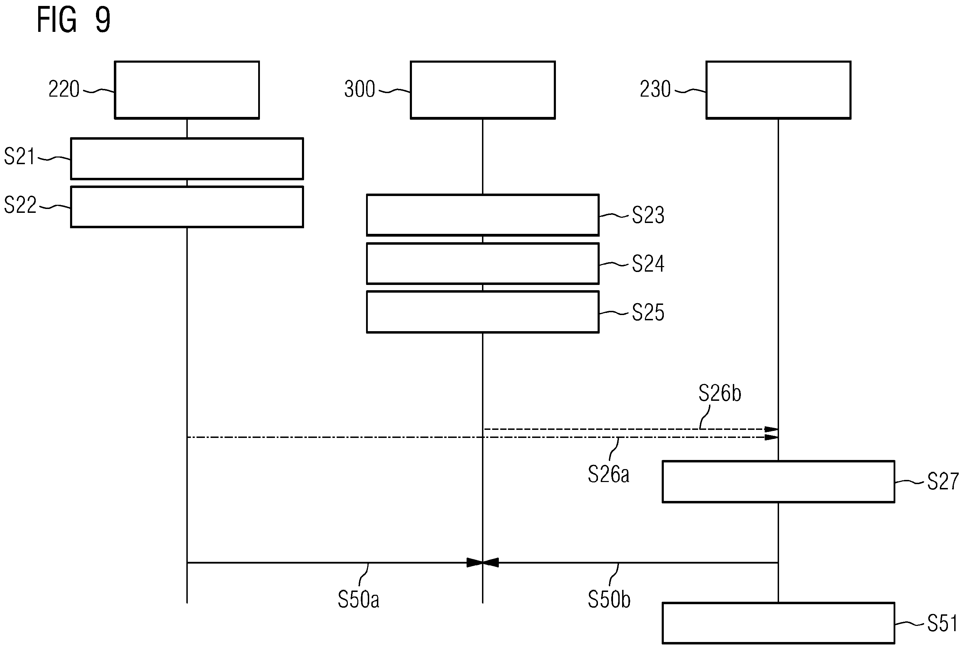

In a first step S11, a health-related application instance is executed on a sending computing device 220. The sending computing device 220 provides means for accessing and controlling the executed health-related application. In a further step S12, an application state of an executed health-related application instance is bookmarked on the sending computing device 220 to be output and shared within the group of collaborating computing devices. In a further step S13, a backend computing device 300 stores the bookmarked application state of an application instance. The health-related application instance can be executed on the sending computing device 220. In a further step S14, by the backend computing device 300, a container as a data structure including health-related application data is generated on the basis of the stored bookmarked application state. In a further step S15, a link referring to the container is generated by the backend computing device 300. In a further step, a sending of the generated link to at least one receiving computing device of the collaborating computing devices is initiated. The initiation can be processed by the backend computing device 300 in step S16b (see Fig. 9) or in step S16a (see Fig. 9) by the sending computing device. In a further step S17, the link is received by the at least one receiving computing devices 230. In an embodiment, a plurality of computing devices 230 can be selected and the link can be received by the selected plurality of computing devices 230. In a further step S18, the received link is activated by the at least one receiving computing device 230. By activating the link, access to the health-related application data is provided and thereby processing an instance of the container. In a further step S19, any processed instance of the container is stored by the backend computing device 300. Further, any processed instance of the container is made available for further collaboration within the group of collaborating devices.

Fig. 5 shows a further flowchart of a method 20 for digital cooperation for a group of collaborating computing devices 200 by providing access to a health-related application persisting an application state for a medical assessment according to an embodiment of the invention. In the illustrated exemplary embodiment according to Fig. 5, method 20 comprises several steps.

In a first step S21, a health-related application instance providing means for accessing and controlling the executed health-related application is executed by a sending computing device 220. In a further step S22, an application state of the accessed health-related application on the sending computing device 220 to be output and shared within the group of collaborating computing devices is bookmarked by the sending computing device 220. In a further step S23, by a backend computing device 300 the bookmarked application state of an application instance has been stored. The health-related application instance is executed on the sending computing device 220. In a further step S24, by the backend computing device 300, a container as a data structure including health-related application data is generated on the basis of the stored bookmarked application state. In a further step S25, a link referring to the generated container has been generated by the backend computing device 300. In a further step S26 a sending of the generated link to at least one receiving computing device 230 of the collaborating computing devices 200 is initiated. In an embodiment, in step S26a, the sending of the generated link can be initiated by the sending computing device 220. In a further embodiment, the sending of the generated link can be initiated in step S26b by the backend computing device 300. In a further step S27, access for at least one further selected computing device 230 to a collaboration application configured for data processing of the application state in the container and thereby processing an instance of the container is provided.

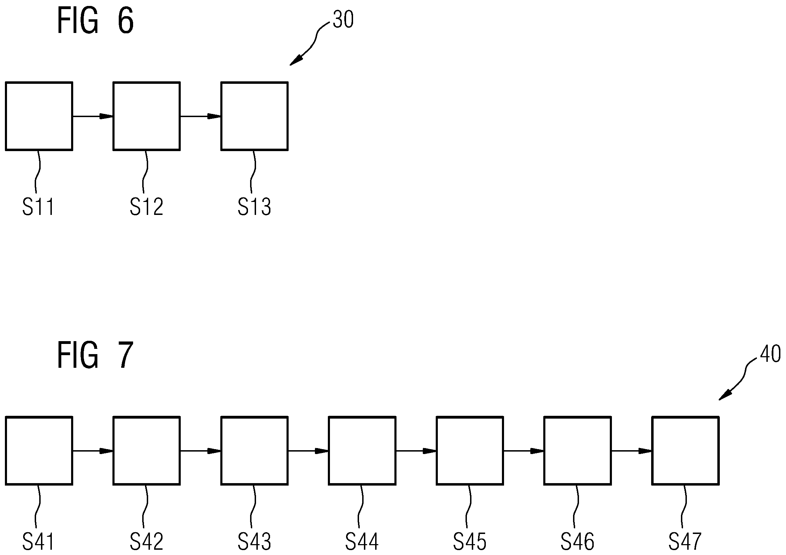

Fig. 6 shows a further flowchart of a method 30 for digital cooperation for a group of collaborating computing devices 200 by providing access to a health-related application persisting an application state for a medical assessment according to an embodiment of the invention. In the illustrated exemplary embodiment according to Fig. 6, method 30 comprises several steps.

In a first step S31, a link referring to a container as a data structure including health-related application data is received by at least one further selected receiving computing device 230. In a further step S32, the container is accessed by the at least one further selected receiving computing device 230 the container by processing the received link. In a further step S33, access to a collaboration application configured for data processing of the application state in the container and thereby processing an instance of the container is received by the at least one receiving computing device 230.

Fig. 7 shows a further flowchart of a method 40 for digital cooperation for a group of collaborating computing devices 200 by providing access to a health-related application persisting an application state for a medical assessment according to an embodiment of the invention. In the illustrated exemplary embodiment according to Fig. 7, method 40 comprises several steps, which are executed on the backend computing device 300.

In a first step S41, a bookmarked application state of an application instance is received. The application instance is executed on a sending computing device 220. In a further step S42, bookmarking signals from a sending computing device 220 for bookmarking an application state of the accessed health-related application on the sending computing device 220 to be output and shared within the group of collaborating computing devices are received. In a further step S43, a bookmarked application state of an application instance being executed on the sending computing device 220 is stored. In a further step S44, a container as a data structure including health-related application data is generated on the basis of the stored bookmarked application state. In a further step S45, a link referring to the generated container is generated. In a further step S46, the generated link is provided to the sending computing device 220. In a further step S47, access for at least one further selected computing device (230) to a collaboration application for data processing of the application state in the container and thereby generating a processed instance of the container is proved by the generated link.

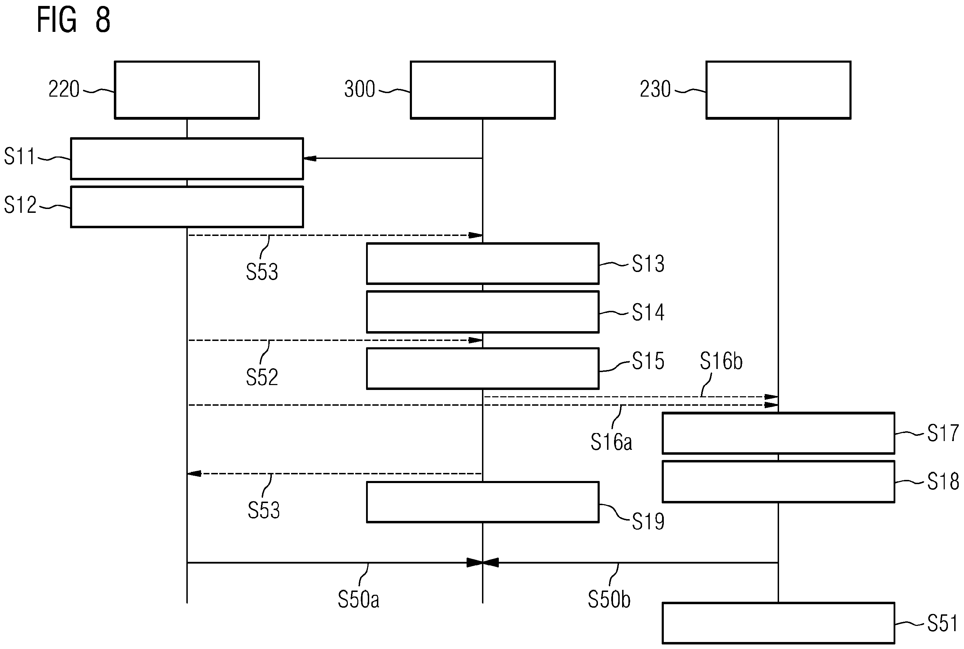

Fig. 8 shows a schematic representation of a time sequence for providing access to a health-related application according to an embodiment of the invention.

In the illustrated exemplary schematic representation of a time sequence according to Fig. 8, a sending computing device 220 is shown. The sending computing device 220 may include a computer, a workstation, a laptop, a smartphone, a tablet, or a smartwatch. The sending computing device 220 comprises a user interface 202 to display information as well as to receive controlling commands by a user. Further, the sending computing device 220 comprises a communication interface 203 for connecting the sending computing device 220 with a backend computing device 300 and a receiving computing device 230. The sending computing device 220 further comprises a processing unit 201 for processing program application code as well as a health-related application instance provided (streamed) by the backend computing device 300. In step S11, a health-related application instance is executed on the sending computing device 220. The sending computing device 220 provides means (e.g., keyboard, mouse, stick, touchscreen, etc.) for accessing and/or controlling the executed health-related application instance. The health-related application instance can be a stream provided by the backend computing device 300. The backend computing device 300 processes a health-related application and provides for each computing device 200 (see Fig. 1) connected to the backend computing device 300 an instance as stream of the health-related application.

The health-related application instance as streamed to the sending computing device 220 in step S11 includes health-related information, e.g., set of images of an image scanner including notes, annotations, markings, further documents that can be used for a further medical examinations or treatment. The streamed health-related application instance can be opened within a browser in a viewer window/application. The stream is processed by the processing unit 201 of the sending computing device 220 and output in the browser.

The user can use the user interface 202 at the sending computing device 220 to bookmark an application state of the in step S11 executed health-related application instance processed on the sending computing device 220. In step S12 the bookmarked health-related application instance is generated. The application state includes a snapshot of the parts of the processed health-related application instance that the user wants to share with the bookmarking. The bookmarked application state of the health-related application instance on the sending computing device 220 can be output and shared within the group of collaborating computing devices 220 including further sending computing devices 220x and receiving computing devices 230x.

The executed bookmarked health-related application instance of the health-related application is stored in step S13 by the backend computing device 300. The backend computing device 300 comprises a communication interface 302 for connecting with a communication connection 101. Further, the backend computing device 300 comprises a distributed storage 303. The distributed storage 303 can be embedded in the backend computing device 300 or outsource in an external storage connected to the backend computing device. The distributed storage 303 is used to store at least one bookmarked application state. In an embodiment, the bookmarking of an application state comprises generating a bookmark in step S53 pointing to the application instance of the bookmarked application state by using the means provided with the sending computing device 220.

The backend computing device 300 further comprises a processing unit 301 to generate in step S14 on the basis of the stored bookmarked application state a container as a data structure including health-related application data. The data structures can be designed using JSON or XML. The health-related application data may comprise application features of the executed health-related application instance, the application state, enriched with meta information, accessed and bookmarked health-related data, references to literature, in particular scientific literature, medical knowledge associated to the case to be collaborated on in particular in an encoded form, chat data, in particular chat history data and/or a unique identifier associated to the generated container.

The backend computing device 300 further generates in step S15 a link referring to the generated container. In an embodiment, generating the link referring to the generated container is executed after receiving in step S52 a request signal issued by the sending computing device 220. Thus, the sending computing device 220 controls the sharing of the bookmark health-related application instance. Moreover, the link can be shared with a (further) receiving computing device 230 via a chat function. The link is pointing to a storage area in the distributed storage 303 storing the generated container. The link can be designed as an URL link including a specific reference pointing to the container. The reference is a unique reference used to identify the container. The reference can be a random generated reference consisting of digits, letters, and signs, as well as of a combination of digits, letters, and signs.

In the following step S16b, the backend computing device 300 initiates a sending of the generated link to at least one receiving computing device 230 of the collaborating computing device 200. In an alternative embodiment, step S16a includes initiating a sending of the generated link to at least one further receiving computing device 230 of the collaborating computing devices by the sending computing device 200.

In step S17, the link is received by the at least one further selected computing device 230 via, for instance, a chat function. The received link is processed by the selected computing device 230. In the following step S18, the received link is activated by at least one further selected computing device 230. According to the choice of the sending computing device 222, the link can be sent to multiple receiving computing devices 230x and activated. The received link provides access to the health-related application data and thereby processing an instance of the container. In particular, the processing unit 301 of the receiving computing device 300 processes an instance of the health-related application bookmarked by the sending computing device using the bookmarked application state. The backend computing device 300 stores in a step S19 any processed instance of the container and making it available for further collaboration within the group of collaboration computing devices 200. In particular, the backend computing device 300 stores each instance where a sending computing device 220 and/or a receiving computing device has made changes, e.g., adding new annotations, adding further clinical data from a different medical field, adding notes etc. Each of the stored instances can be shared by the creator to any of the computing device of the collaborating computing devices 200.

In a further embodiment, it can be required that each of the computing devices 220x, 230x has to authenticate itself before entering the system 100 and start communicating with each other or with the backend computing device 300. In steps S50a, S50b, the collaborating computing devices are authenticated versus the backend computing device 300. In a further embodiment it is intended that the receiving computing device 230 has to be authenticated before receiving the link. If the authentication was successful and the receiving computing device 230 is intended for access, in step S51, access for the authenticated computing device 230 to the container by activating the received link is provided. For the authentication different methods can be used including the knowledge, possession, biometrical features approach. Further not listed approaches as well as a combination of these approaches can be used for authentication of the computing devices 200.

Fig. 9 is a further schematic representation of a time sequence for providing access to a health-related application according to a further embodiment of the invention.

In the illustrated exemplary schematic representation of a time sequence according to Fig. 9, a sending computing device 220, a backend computing device 300, and a receiving computing device 230 are shown.

The sending computing device 220 executes in a step S21 a health-related application instance. The sending computing device 220 provides means for accessing and controlling the executed health-related application instance. In step S22 the sending computing device 220 bookmarks an application state of the accessed health-related application on the sending computing device 220 to be output and shared within the group of collaborating computing devices 200. The bookmarked application state is provided to the backend computing device 300. In step S23 the bookmarked application state of the application instance executed on the sending computing device 220 is stored at the backend computing device 300. In particular in the distributed storage 303 of the backend computing device 300. The distributed storage 303 can be included in the backend computing device 300. In an alternative embodiment is the distributed storage 303 excluded and in communication connection with the backend computing device 300.

In a further step S24, a container as a data structure including health-related application data is generated by the backend computing device 300 on the basis of the stored bookmarked application state. Further, a link referring to the generated container is generated in step S25 by the backend computing device 300. By the backend computing device 300 in step S26b or alternatively in step S26a the sending of the generated link to at least one receiving computing device 230 of the collaborating computing devices 200 is initiated. Further, in step S27 access is provided by the sent link for at least one receiving computing device 230 to a collaboration application configured for data processing of the application state in the container and thereby processing an instance of the container.

In a further embodiment, an authentication of the involved sending computing devices 220x and receiving computing device 230 is performed in steps S50a, S50b, S51. In particular, the receiving computing device 230 has to be authenticated before receiving and activating the link provided by the sending computing device 220 or the backend computing device 300. In this way, unauthorized access to protected data can be prevented.

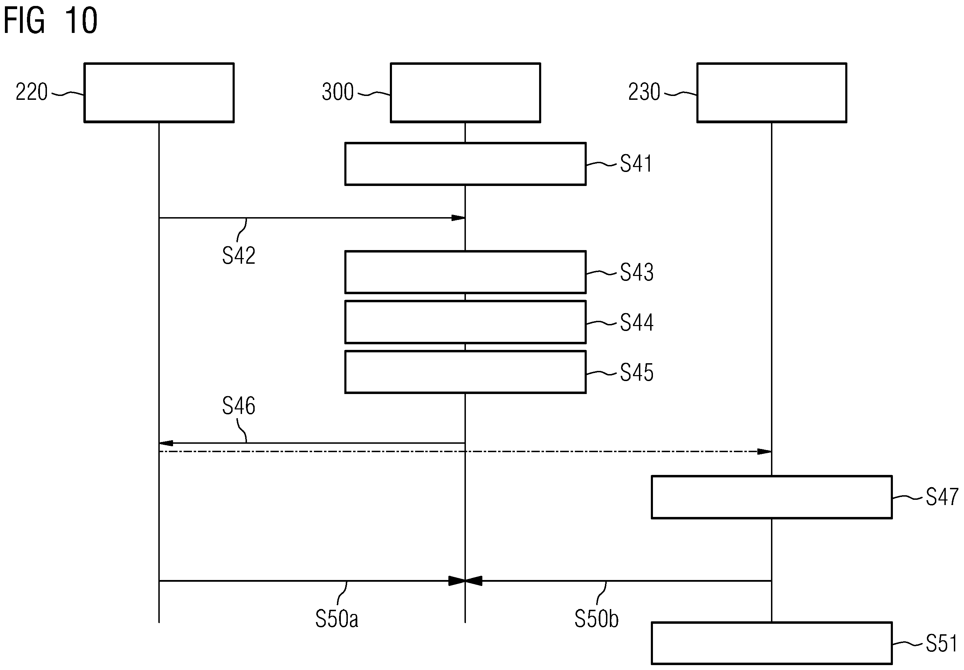

Fig. 10 is a further schematic representation of a time sequence for providing access to a health-related application according to a further embodiment of the invention. In the illustrated exemplary schematic representation of a time sequence according to Fig. 10, a sending computing device 220, a backend computing device 300, and a receiving computing device 230 are shown.

In a step 41, the backend computing device 300 executes a health-related application that is processed by the sending computing device 220 as a health-related application instance. From the sending computing device 220, the backend computing device 300 receives a bookmarked application state of the health-related application instance. The health-related application instance is an instance of a health-related application executed by the processing unit 301 of the backend computing device 300 and streamed to be processed and output by the sending computing device 220. A user using the sending computing device 220 can make a snapshot of the running health-related application instance referring to a bookmarked application state and trigger a sending of the bookmarked application state of the health-related application instance with related information, he or she wants to share. The related information may be annotations or may be findings in a further application (e.g. laboratory values in addition to images from a MRI scanner). In the following step S42, the backend computing device 300 receives bookmarking signals from the sending computing device 220 for bookmarking an application state of the accessed health-related application on the sending computing device 220 to be output and shared within the group of collaborating computing devices.

The bookmarked application state of the application instance being executed on the sending computing device 220 is stored in step S43 in the distributed storage 303. Further, in step S44 a container as a data structure including health-related application data is generated on the basis of the stored bookmarked application state. In the following step S45, a link referring to the generated container is generated. The link is pointing to a storage area of the distributed storage 303 storing the container. The link includes a unique reference only referring to a specific container itself including the reference.

The generated link can be provided by the backend computing device 300 in step S46 to the sending computing device. The generated link can be shared with any computing device 220x, 230x of the collaborating computing device 200. In the illustrated embodiment of Fig. 10, the link is provided (shared) by the sending computing device 220 (illustrated by the dashed arrow). In a further embodiment, the link can be provided by the backend computing device 300, when triggered by the sending computing device 220. In a further step, access is provided by the received link for at least one further selected computing device 230 to a collaboration application for data processing of the application state in the container and thereby generating a processed instance of the container.

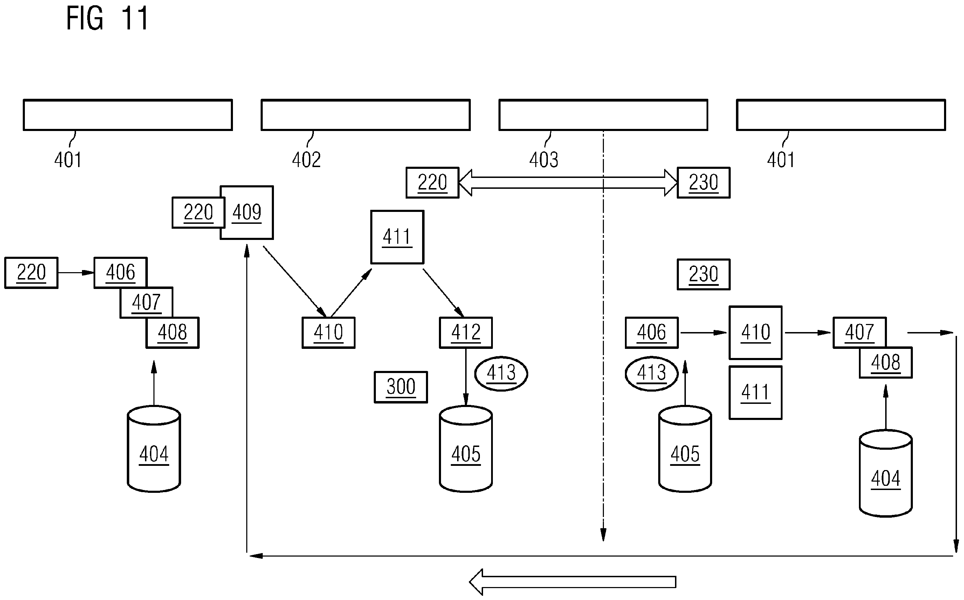

Fig. 11 is a schematic representation of the communication system according to an embodiment of the invention. In the illustrated exemplary schematic representation of the communication system according to Fig. 11, an information access layer 401, an information model layer 402, and a communication layer 403 are shown.

In an embodiment, the system comprises at least an information access layer (IAL) 401. The information access layer 401 is configured to be the bridging part 407 (access to information layer) between a user interface 202 of the sending computing device 220 and the several information providing systems 404 that are storing and providing specific medical health-related information e.g., DICOM image data, structured report (analysis for a medical diagnoses/treatment) in PACS, unstructured physician's letter in LIS, and/or clinical information in a hospital information system, HIS. Thus, the information access layer provides/includes interfaces for communication with different data sources and to support the data transfer.

Further, the information access layer (IAL) 401 provides functionalities for administration of user specific authentication methods 406 of different systems. For authentication can be used a password authentication, email related authentication, short message service (SMS), applications running on a separate device (app), physical aids including hardware token, biometric features, two-factor authentication and/or single-sign-on.

Further, the information access layer (IAL) normalizes the read information from the several data source to a general patient data model for further usage by the involved physicians in the diagnoses/treatment. The normalization can be performed automatically according to before set parameter settings choses individually by the physician/viewer 408.

Further, the information access layer (IAL) 401 provides an interactive user interface 409 for accessing or query the patient information. The interactive user interface that is configured to display communicated aggregated information content in a way that is appropriate for the receiving computing device 230. The information access layer 401 allows the information to be viewed in various depths of detail. Further, the information layer 401 allows additional content that is not primarily prepared to be viewed and to be communicated back as a feedback to any question the communication partner may have, together with textual or verbal descriptions. The user interface can be used on various devices (e.g., work station, computer, laptop, smartphone, tablet, smartwatch, beamer, etc.) and allows both synchronous and asynchronous communication.

In a further embodiment, the system comprises at least an information model layer 402. The information model layer 402 receives the output of the information access layer 410. The output of the information access layer 401 includes at least an application state of a health-related application instance process on a sending computing device 220. Different information or information fragments (specific document, values from the document, etc.) are combined into a semantic state, in particular in an application state automatically by using different wizards or by user interaction including a manual selection. The processes executed are represented as event chains. An event chain is listing of events. An event can be related to other events, which will create event chains. These event chains can significantly affect the course of the processes. Events can be correlated with each other without one triggering another one. The fragments of the event chain are represented in the cache. This result in a faster and more efficient access to the cache memory, when new and similar event chains are triggered.

The information model layer 402 is used to map time-critical information, persistent information, and/or process results in the cache. A specific view including a processed instance is stored and provided by using the type of data as used, the semantic link between the different types of data as well as the knowledge about the user's field of expertise and the meaning of the data. The output of the information model layer 402 is a semantic state 410 of an executed health-related application instance. The semantic state 410 may include opened files, data specific states, markings, notes, zooming information, etc. The data attached to the semantic state 410 may include data stored in different data storages 405 provided by different medical experts. Further, the sematic state 410 (application state) can be stored in the storages 405. In an embodiment, the storage 405 is included in the backend computing device 300. The bookmarked application state is stored in a container in the data storage 405 including a specific and unique reference 413. Using the specific and unique reference 413, the container can be found and identified by the link generated by the backend system 300. The semantic state 410 is provided by the container.

In a further embodiment, the system comprises at least a communication layer 403. The communication layer (CL) 403 is the layer used for bookmarking an application state of an executed health-related application instance. For generating the bookmarked health-related application instance is the output (result) of the information model layer 402 including the semantic state 410. The communication layer 403 has access to annotations/augmentations 411 of the semantic state 410, which cannot be extracted from the patient data sources, in particular are no part of the sources and has been added later on in particular assessments. The semantic state 410 and the annotations/augmentations 411 are stored by the communication layer 403 in specific databases, e.g., a bookmark database 405 together with further meta information, e.g., creator of the data, date of creation, specific tag, etc. The bookmark database 405 is a system for electronical data administration configured to store a large quantity of data efficiently, without contradiction, and permanently. The bookmark database 405 may be used to bundle different bookmarks in containers with additional meta information. Each stored bookmark may be identified with a random generated but unique identifier 413. The identifier 413 can be used for a synchronous communication (arrow directing in both directions between the sending computing device 220 and the receiving computing device 230) including a chat inside a bookmark as well as for an asynchronous communication (feedback line from 408 to 409) including loading a bookmark, changing the bookmark and thus creating a new, associated bookmark, and saving both bookmarks in a container. In this way, a new container is generated including the information of the previous container as well as the new data added. A new container is stored in the database 405. For this container a new link can be generated and shared within the collaboration of computing devices 220.

Further, the communication provides the reference to the stored bookmark in the database 405 that can be used to access the database for usage. The access be a receiving computing device 230 is organized and managed by the information access layer. Using the reference, the sematic state 410 can be loaded to the cache memory. Further, stored annotations/augmentations 411 as well as meta information (creator, associated container) are loaded from the database 404 and can be output for a receiving computing device 230.

Wherever not already described explicitly, individual embodiments, or their individual aspects and features, described in relation to the drawings can be combined or exchanged with one another without limiting or widening the scope of the described invention, whenever such a combination or exchange is meaningful and in the sense of this invention.

Advantages which are described with respect to a particular embodiment of present invention or with respect to a particular figure are, wherever applicable, also advantages of other embodiments of the present invention.

## Claims

1. Method (10) for digital cooperation for a group of collaborating computing devices by providing access to a health-related application persisting an application state for a medical assessment, comprising the method steps of:
- Executing (S11) a health-related application instance on a sending computing device (220) providing means for accessing and controlling the executed health-related application;
- Bookmarking (S12) an application state of an executed health-related application instance on the sending computing device (220) to be output and shared within the group of collaborating computing devices;
- Storing (S13) by a backend computing device (300) the bookmarked application state of an application instance, the application instance being executed on the sending computing device (220);
- Generating (S14) by the backend computing device (300) on the basis of the stored bookmarked application state a container as a data structure including health-related application data;
- Generating (S15) by the backend computing device (300) a link referring to the generated container;
- By the backend computing device (300) or by the sending computing device (220): Initiating (S16a, S16b) a sending of the generated link to at least one receiving computing device (230) of the collaborating computing devices; and
- Receiving (S17) the link by the at least one receiving computing device (230);
- Activating (S18) the received link by the at least one receiving computing device (230) for providing access to the health-related application data and thereby processing an instance of the container; and
- Storing (S19) by the backend computing device (300) any processed instance of the container and making it available for further collaboration within the group of collaborating devices.

2. Method (20) for digital cooperation for a group of collaborating computing devices by providing access to a health-related application persisting an application state for a medical assessment, comprising the method steps of:
- By a sending computing device (220): Executing (S21) a health-related application instance providing means for accessing and controlling the executed health-related application;
- By the sending computing device (220): Bookmarking (S22) an application state of the accessed or executed health-related application on the sending computing device (220) to be output and shared within the group of collaborating computing devices;
- By a backend computing device (300): Storing (S23) the bookmarked application state of an application instance, wherein the application instance is executed on the sending computing device (220);
- By the backend computing device (300): Generating (S24) on the basis of the stored bookmarked application state a container as a data structure including health-related application data;
- By the backend computing device (300): Generating (S25) a link referring to the generated container;
- By the sending computing device (220) or by the backend computing device (330): Initiating (S26a, 26b) a sending of the generated link to at least one receiving computing device (230) of the collaborating computing devices;
- Providing (S27) by the sent link access for at least one receiving computing device (230) to the health-related application configured for data processing of the application state in the container and thereby processing an instance of the container.

3. Method (30) for digital cooperation for a group of collaborating computing devices by providing access to a health-related application persisting an application state for a medical assessment, comprising the method steps of:
- Receiving (S31) by at least one receiving computing device (230) a link referring to a container as a data structure including health-related application data;
- Accessing (S32) by the at least one receiving computing device (230) the container by processing the received link; and
- Receiving (S33) by the at least one receiving computing device (230) access to a collaboration application configured for data processing of the application state in the container and thereby processing an instance of the container.

4. Method (40) for digital cooperation for a group of collaborating computing devices by providing access to a health-related application persisting an application state for a medical assessment, comprising the method steps, which are executed on a backend computing device (300):
- Receiving (S42) bookmarking signals, indicating a bookmarked application state of an application instance, being executed on a sending computing device (220) from the sending computing device (220) to be output and shared within the group of collaborating computing devices;
- Storing (S43) the bookmarked application state of the application instance, being executed on the sending computing device (220);
- Generating (S44) on the basis of the stored bookmarked application state a container as a data structure including health-related application data;
- Generating (S45) a link referring to the generated container;
- Providing (S46) the generated link to the sending computing device (220);
- Via the generated link: Providing (S47) access for at least one receiving computing device (230) to the health-related application for data processing of the application state in the container and thereby generating a processed instance of the container.

5. Method according to the preceding claims 1 or 4, wherein the method further comprises at least the step of:
- Authenticating (S50a, S50b) the collaborating computing device and in particular authenticating the receiving computing device (230); and
- Providing (S51) access for the authenticated collaborating computing devices (220, 230) to the container by activating the received link.

6. Method according to the preceding claims 1 or 4, wherein generating by the backend computing device (300) the link referring to the generated container is executed after receiving (S52) a request signal issued by the sending computing device (220).

7. Method according to claims 1 or 2, wherein bookmarking an application state comprises generating (S53) a bookmark pointing to the application instance of the bookmarked application state by using the means provided with the sending computing device (220).

8. Method according to any of the preceding claims, wherein the health-related application data at least comprises:
- application features of the executed health-related application instance;
- the application state, enriched with meta information;
- accessed and bookmarked health-related data;
- references to literature, in particular scientific literature;
- medical knowledge associated to the case to be collaborated on in particular in an encoded form;
- chat data, in particular chat history data and/or
- a unique identifier associated to the generated container.

9. Method according to any of the preceding claims, wherein the link is generated according to a communication system for processing the link and includes at least a reference to an allocated memory storing the container.

10. Method according to any of the preceding claims 1 or 2, wherein executing a health-related application comprises aggregating data from different heterogeneous data sources and linking the aggregated data to the container using the meta information and/or executing related additional applications, accessible via the health-related application.

11. Method according to any of the preceding claims, wherein the collaboration application provides means for exchanging reference cases for the collaboration case.

12. Method according to any of the preceding claims, wherein the collaboration application provides means for annotating and/or processing the health-related application and/or data of the health-related application.

13. Method according to any of the preceding claims, wherein a sending computing device (220), which has requested the link and a receiving computing device (230), which has received the link are positioned in different software and hardware environments with different viewing applications.

14. System (100) for digital cooperation for a group of collaborating computing devices by providing access to a health-related application persisting an application state for a medical assessment, comprising:
- A set of collaborating computing devices (200), each comprising a processing unit (201), a user interface (202), and a communication interface (203), wherein at least one collaborating computing device (200) acts as a sending computing device (220) and at least another one acts as a receiving computing device (230) and still other acts as a backend computing device (300);
- Distributed storages (303) for storing a health-related application and in particular different instances of the health-related application, which may be executed by the processing unit (201) of the collaborating computing devices (200);
- wherein the user interface (202) of the sending computing device (220) is configured for selecting an application state of the executed health-related application instance;
- wherein the computing device (200) acting as the sending computing device (220) is configured for:
- Executing and/or controlling a health-related application instance;
- Bookmarking an application state of the executed health-related application instance, wherein the application state is to be output and shared within the group of collaborating computing devices; and
- Initiating a sending of a generated link to at least one receiving computing device of the collaborating computing devices for activating the sent link by the at least one receiving computing device for providing access to the health-related application data and thereby processing an instance of the container; and
- wherein the computing device (200) acting as a backend computing device (300) is configured for:
- Storing the bookmarked application state of an application instance, being executed on the sending computing device;
- Generating on the basis of the stored bookmarked application state a container as a data structure including health-related application data;
- Generating the link referring to the generated container; and
- Storing any processed instance of the container and making it available for further collaboration within the group of collaborating devices.

15. System according to the directly preceding claim, wherein the system (100) is further configured for:
- authenticating the computing devices (200) and in particular authenticating the receiving computing device (230) ;
- providing access for the authenticated receiving computing devices (200, 220, 230) to the container by activating the received link.

16. A computer program product (500) comprising a computer program (501), the computer program (501) being loadable into a memory unit of a computing unit, including program code sections to make the computing unit execute the method for digital cooperation according to any of the method claims above, when the computer program (501) is executed in said computing unit.
